# EUROPEAN PATENT SPECIFICATION

(11) **EP 1 242 092 B1**
(45) Date of publication and mention of the grant of the patent: **02.07.2003**
(21) Application number: 01940311.2
(22) Date of filing: 04.04.2001
(51) Int. Cl.: A61K 31/55, A61K 31/165, A61K 31/19, A61P 29/00

(54) **DRUG COMBINATION FOR THE TREATMENT OF HEADACHE COMPRISING MIRTAZAPINE AND PARACETAMOL OR A NON-STEROIDAL ANTI-INFLAMMATORY DRUG**
ARZNEISTOFFKOMBINATION ZUR BEHANDLUNG VON KOPFSCHMERZEN MIT MIRTAZAPINE UND PARACETAMOL ODER NICHT-STEROIDEN ENTZÜNDUNGSHEMMENDEN MITTELN
COMBINAISON DE MEDICAMENTS DESTINEE AU TRAITEMENT DU MAL DE TETE COMPRENANT MIRTAZAPINE ET UN MEDICAMENT ANTI-INFLAMMATOIRE NON STEROIDE

(30) Priority: 05.04.2000 EP 00201239
(43) Date of publication of application: 25.09.2002
(73) Proprietor: Akzo Nobel N.V., 6824 BM Arnhem (NL)
(72) Inventor: NICKOLSON, Victor Johannes, NL-5345 JB Oss (NL)
(74) Representative: Kraak, Hajo
(86) International application number: EP0104069
(87) International publication number: WO01074340

(56) References cited:
- WO-A-98/19674

## Description

The invention relates to a combination comprising paracetamol or a non-steroidal anti-inflammatory drug (NSAID), or a pharmaceutically acceptable salt or solvate thereof, and another drug, to a patient pack containing dose units comprising paracetamol or an NSAID or mirtazapine, and to a method of treatment of headache and related complaints.

Headache is a major cause of discomfort and a cause of a considerable amount of lost capacity for daily functioning. Many drugs are available to combat the complaint. Headache, in its severest form, is part of the complex of symptoms defining migraine. Other forms of headache occur episodically or periodically during menses or due to stress. The latter is known as tension-type headache. Analgesics, such as paracetamol, or non-steroidal anti-inflammatory drugs are usually used to combat the complaint. On many occurrences of headache the use of one drug is not sufficient and recourse is made to combinations of drugs in the field. For migraine such combinations are with analgesics, non-steroidal anti-inflammatory drugs (NSAID's), ergot alkaloids, antiserotonergics and anti-histamines. These combinations can have benefit due to additive effects of the components of the combination. More desirable is a truly synergistic effect of two drugs in the sense that the effect of the combination is superior over an additive effect of the effects of both drugs in an individual patient. There are only very few synergistic therapeutic drug interactions known which have found acceptance in the area of treatment of headache. Finding more effective drug therapies and more effective combinations is therefore an ongoing endeavour. It is the object of this invention to improve the means for treatment of headache and more specifically the means for treatment of tension-type headache. It is a further object of this invention to enhance the efficacy of common analgesics and an NSAID for the treatment tension-type headache.

Although combinations of paracetamol or NSAID's with other drugs are known (see for example van Gerven et al. British J Clinical Pharmacology volume 41, pages 475-481, 1996), it is found now that combining paracetamol or an NSAID with the antidepressant drug mirtazapine has an extremely favourable effect in the treatment of headache.

This invention provides for a combination according to the opening paragraph, in which combination the other drug is mirtazapine, or a pharmaceutically acceptable salt or solvate thereof, optionally in association with one or more pharmaceutically acceptable carriers. More specifically, the invention provides for a combination comprising an amount of mirtazapine, or a pharmaceutically acceptable salt or solvate thereof, and an amount of paracetamol or an NSAID, or a pharmaceutically acceptable salt or solvate thereof, optionally in association with one or more pharmaceutically acceptable carriers, whereby the amount of paracetamol or the NSAID and the amount of mirtazapine are such that the effect of the combination is more favourable than the added effects of the amounts of each drug separately. Thus, the combination of paracetamol or an NSAID with mirtazapine truly has a synergistic interaction when used in the treatment of headache. As a consequence, the combined use of mirtazapine and an NSAID or paracetamol has better effects in more patients in comparison with the use of paracetamol or an NSAID alone. The better effect can reside in fewer side effects or a faster or more complete recovery in individual patients or in the overall result of the treatment of a group of patients. The preferred use of the combination will be in the treatment of tension-type headache.

Mirtazapine (Org 3770; disclosed in US patent 4,062,848) is a modern drug for the treatment of depression and anxiety with favourable side effect profiles and very low risks for a lethal overdose. For mention of mirtazapine in the context of treatment of headache see WO 98/19674. Non-steroidal anti-inflammatory drugs, which are the preferred drugs for combining with mirtazapine, are known from different chemical classes such as salicylics, pyrazolones or arylpropionics. Examples of specifically known NSAID are: aceclofenac, antipyrine, aspirin (acetylsalicylic acid), benoxaprofen, butibufen, carprofen, celecoxib, diclofenac, dipyrone, etodolac, flosulide, flurbiprofen, FR 140423, ibufenac, ibuprofen, indomethacin, ketoprofen, ketorolac, lornoxicam, loxoprofen, lysine clonixinate, M-5011, meclofenamic acid, meloxicam, metiazinic acid, nabumetone, naproxen, NS-398, numesulide, oxyphenbutazone, D-penicillamine, phenylbutazone, piroxicam, pyrazolone, rofecoxib, salsalate, salicylate, SC-58236, SC-58560, sulfasalasine, sulindac, tiaprofenic acid, tenidap, tenoxicam, tepoxalin, tolfenamic acid, tolmetin and zaltoprofen. All these NSAIDS can be used in combination with mirtazapine with the desired improvement of effect. The combination of ibuprofen with mirtazapine is a more preferred combination of the invention.

NSAIDS are supposed to act as inhibitors of the enzyme cyclooxygenase (COX), involved in the biosynthesis of prostaglandins. There are two isoenzymes discovered, indicated as COX-1 and COX-2. It is the enzyme COX-2 that is mostly expressed in tissues with an inflammatory response. Those NSAID's, that inhibit COX-2 more selectively, are presumed to have fewer side effects in the general system, in particular the stomach. The NSAID's more selectively inhibiting COX-2 are carprofen, celecoxib, etodolac, flosulide, nabumetone, numesulide, rofecoxib and SC-58236.

Thus, the present invention concerns the administration of two different drugs from different pharmacological categories, one drug enhancing the therapeutic efficacy of the other drug in the treatment of headache.

It will be appreciated that mirtazapine and the NSAID's and the salts thereof may contain one or more centres of chirality and exist as stereoisomers including diastereomers and enantiomers. The present invention includes the aforementioned stereoisomers within its scope and each of the individual (R) and (S) enantiomers of the compounds and their salts, substantially free, i.e. associated with less than 5%, preferably less than 2%, in particular less than 1% of the other enantiomer and mixtures of such enantiomers in any proportions including racemic mixtures containing substantially equal amounts of the enantiomers.

The following specifications of the terms used above serve to clarify better what is provided by this invention.

The drug name mirtazapine also refers to the individual (R) and (S) enantiomers of mirtazapine. These can be used as their salts, substantially free of the other enantiomer or as mixtures of such enantiomers in any proportions.

Unless otherwise stated all amounts of the active components refer to the weights of mirtazapine as base or of paracetamol or the NSAID's as base or acid without inclusion of an amount of crystal water or added acid or base to form an addition salt. According to the terminology in this description paracetamol or the NSAID's, as at least one component, and mirtazapine, as at least a second component, are the active ingredients or active components of the combination.

Pharmaceutically acceptable addition salts are those that can be obtained with, for example, hydrochloric, fumaric, maleic, citric, succinic acid or sodium or potassium hydroxyde.

The terms pharmaceutically acceptable carriers and excipients refer to those substances known in the art to be allowable as filler or carrier material in pills, tablets, capsules etc. The substances are usually approved for this purpose by health-care authorities and are inactive as pharmacological agents. A compilation of pharmaceutically acceptable carriers and excipients can be found in the Handbook of Pharmaceutical excipients (2^{nd} edition edited by A. Wade and P.J. Weller; Published by the American Pharmaceutical Association, Washington and The Pharmaceutical Press, London in 1994). Specifically, lactose, starch, cellulose derivatives and the like, or mixtures thereof, can be used as carriers for the active components of the combination according to this invention.

The term combination refers to any presentation form in which the intention for use of mirtazapine in combination with paracetamol or an NSAID can be recognised. Such combinations with mirtazapine may in this description also be referred to as combinations according to the invention.
It will be appreciated that the compounds of the combination may be administered concomitantly, either in the same or different pharmaceutical formulation or sequentially. If there is sequential administration, the delay in administering the second (or additional) active ingredient should not be such as to lose the benefit of the efficacious effect of the combination of the active ingredients. A minimum requirement for a combination according to this description is that the combination should be intended for combined use with the benefit of the efficacious effect of the combination of the active ingredients. The intended use of a combination can be inferred by facilities, provisions, adaptations and/or other means to help using the combination according to the invention. For example, a combination can be made suitable by adding instructions or aids or even determinants for the combined use. Determinants for the combined use can, for example, reside in the properties of a dispenser of dosage units of the active ingredients of the combination. The active ingredients can thus be in separate dosage units, but still the combination can have a determinant inducing the use of the dosage units of the combination in a predetermined sequence and/or at pre-determined times by the properties of the dispenser. A preferred determinant for combined use is of course the formulation of both the active components of the combination in one pharmaceutical composition.
Thus according to one aspect, the present invention provides a pharmaceutical composition, comprising mirtazapine, or a pharmaceutically acceptable salt or solvate thereof, and comprising paracetamol or an NSAID, or a pharmaceutically acceptable salt or solvate thereof.

There are several types of headache complaints in people. Tension-type headache is the most common one among this group of complaints. The characteristic is that the pain is typically pressing or tightening in quality, of mild to moderate intensity, bilateral in location and does not worsen with routine physical activity. Nausea is absent, but bothersome hypersensitivity to light and noise may occur. Tension-type headache is preferably selected to be treated with the combination according to this invention. There are two types of tension-type headaches: Episodic tension-type headache and chronic tension-type headache. The first is characterised by recurrent episodes of headache lasting minutes to days. Chronic tension-type headache is characterised by presence for at least 15 days per month for at least 6 months.

The subject amenable for a treatment made available by this invention is a human person. Men and women often respond differently to drug treatment and suffer differently in nature, frequency and severity from headache. Also, there are differences in treatment methods for persons with headache among different age groups. The elderly, adolescents or postmenopausal age groups have different needs for treatment. Such differential factors are to be taken into account in selecting the treatment of this invention and in selecting the exact dose of the active ingredients for the treatment.

For the use of the combination of the present invention it should provide the active ingredients such that effective amounts for treatment are made available. The amount of a combination of mirtazapine (or a pharmaceutically acceptable salt or solvate thereof) and paracetamol or an NSAID (or a pharmaceutically acceptable salt or solvate thereof), required to produce the efficacious effects will, of course, vary and is ultimately at the discretion of the medical practitioner. The factors to be considered include the route of administration and nature of the formulation, the recipient's body weight, age and general condition and the nature and severity of the headache to be treated. In general, parenteral administration requires lower dosages than other methods of administration, which are more dependent upon absorption.

Dosages of paracetamol or NSAIDS for carrying out the invention are in the range generally recommended a particular compound. For example, for ibuprofen the recommended dose is within the range between 10 and 400 mg per recipient per day, with a dosage in the range between 100 and 300 mg as the more preferred range within which to select a dose for the combination. The recipient is the subject receiving the active ingredients of the combination for treatment of headache.

Dosages for mirtazapine generally will be within the range of 0.1 to 60 mg per recipient per day. However, the daily dosages to a recipient are preferably between 0.1 and 10 mg and more preferably lower than 5 mg.

Since the dose of mirtazapine suitable for obtaining the synergistic effect with paracetamol or an NSAID on headache is far below the amount in currently available dosing units, it is another aspect of the invention that it discloses the use of mirtazapine for the manufacture of a medicament for the treatment of headache, which treatment comprises the administration of mirtazapine with a unit treatment dose comprising more than 0.1 and less than 5 mg mirtazapine, which in other words is a unit treatment dose (within the range) between 0.1 and 5 mg mirtazapine.

While it is possible for the active ingredients of the combination to be administered as the raw chemical it is preferable to present them as a pharmaceutical composition, also referred to in this context as pharmaceutical formulation. Suitable compositions include those suitable for oral, rectal, nasal, topical (including transdermal, buccal and sublingual), vaginal or parenteral (including subcutaneous, intramuscular, intravenous and intradermal) administration. Pharmaceutical compositions according to the present invention comprise mirtazapine or paracetamol or an NSAID or a combination thereof comprising mirtazapine, together with one or more pharmaceutically acceptable carriers or excipients and optionally other therapeutic agents. The present invention further provides compositions according to the invention for use in therapy of headache. Furthermore, the invention includes the use of mirtazapine and paracetamol or an NSAID in the manufacture of a medicament comprising mirtazapine and paracetamol or an NSAID with improved efficacy for therapy of headache. This medicament has an enhanced effect or fewer side effects in comparison to each drug alone. The preferred use of the medicament will be for the treatment of tension-type headache. The invention includes as well the use of mirtazapine and paracetamol or an NSAID in the manufacture of medicaments for administration in combination (either concomitantly or sequentially) respectively with paracetamol or an NSAID, or with mirtazapine, for the treatment of headache.

An important aspect of the present invention is that it provides a method for the treatment of an individual of a vertebrate species, for example, a mammal including a human patient, suffering from headache, which method of treatment comprises administering an effective amount of mirtazapine in combination with paracetamol or an NSAID. The desired daily doses for a treatment is preferably presented as a single dose or in two, or three sub-doses administered at appropriate intervals throughout the day. In practice this means among others to provide dosage units comprising mirtazapine and dosage units comprising paracetamol or an NSAID in a combination or to provide dosage units comprising mirtazapine and paracetamol or an NSAID for administration to a recipient or intake by a recipient for treatment.
Thus, in one embodiment of the invention a mixture of mirtazapine and paracetamol or an NSAID may be presented as a pharmaceutical formulation in dosage unit form, for example, administered in the form of a tablet, pill, capsule and the like. Such dosage forms are known in the art, e.g. as described in the standard reference, Gennaro et al., Remington's Pharmaceutical Sciences, (18th ed., Mack Publishing Company, 1990, see especially Part 8: Pharmaceutical Preparations and Their Manufacture). By means of pharmaceutically suitable liquids the compounds can also be applied as an injection preparation in the form of a solution, suspension, emulsion, or as a spray, e.g. a nasal spray.

For the preparation of pharmaceutical compositions and more specifically dosing units, the present invention further includes a process for the preparation of a pharmaceutical formulation comprising mirtazapine and paracetamol or an NSAID, which process comprises bringing an amount of mirtazapine (or a pharmaceutically acceptable salt thereof) and amount of paracetamol or an NSAID (or a pharmaceutically acceptable salt thereof) into association with one or more pharmaceutical excipients.

More commonly these days pharmaceutical formulations are prescribed to the patient in "patient packs" containing a number dosing units or other means for administration of metered dose units for use during a distinct treatment period in a single package, usually a blister pack. Patient packs have an advantage over traditional prescriptions, where a pharmacist divides a patients supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the combination treatment of headache can be inferred by instructions, facilities, provisions, adaptations and/or other means to help using the formulation most suitably for the treatment. Such measures make a patient pack specifically suitable for and adapted for use for treatment with the combination of the present invention.

Specifically, a further embodiment includes a package containing separate dose units, one or more of which containing mirtazapine or a pharmaceutically acceptable salt thereof and one or more of which containing paracetamol or an NSAID or a pharmaceutically acceptable salt thereof. A package contains enough tablets, capsules or the like to treat a patient for a pre-determined period of time, for instance for 1 week.
Thus, the invention provides a patient pack for the treatment of headache comprising means for administration of metered dose units in combination with packaging material suitable for said dose units, which patient pack comprises mirtazapine and paracetamol or an NSAID and optionally, said packaging material is including means to help a recipient using the dose units most suitably for the treatment headache. Furthermore, the invention provides such a patient pack for the treatment of headache comprising means for administration of metered dose units in combination with packaging material suitable for said dose units, which dose units comprise pharmaceutical auxiliaries and mirtazapine in an amount between 0.1 and 5 mg and optionally, said packaging material is including means to help a recipient using the dose units most suitably for the treatment headache.

The compositions (formulations) according to this invention may be prepared by any methods well known in the art of pharmacy, for example, using methods such as those described in Gennaro et al., Remington's Pharmaceutical Sciences (18th ed., Mack Publishing Company, 1990, see especially Part 8: Pharmaceutical Preparations and their Manufacture). Such methods include the step of bringing into association an active ingredient with a carrier, which constitutes one or more accessory ingredients. Such accessory ingredients include those conventional in the art, such as, fillers, binders, diluents, disintegrants, lubricants, colorants, flavouring agents and wetting agents.

Formulations suitable for oral administration may be presented as discrete units such as pills, tablets or capsules each containing a predetermined amount of active ingredient(s); as a powder or granules; as a solution or suspension. The active ingredient(s) may also be present as a bolus or paste, or may be contained within liposomes.

Formulations for rectal administration may be presented as a suppository or enema.

For parenteral administration, suitable formulations include aqueous and non-aqueous sterile injection. The formulations may be presented in unit-dose or multi-dose containers, for example, sealed vials and ampoules, and may be stored in a freeze dried (lyophilised) condition requiring only the addition of the sterile liquid carrier, for example, water prior to use.

Formulations suitable for administration by nasal inhalation include fine dusts or mists, which may be generated by means of metered, dose pressurised aerosols, nebulisers or insufflators.

For making dose units, e.g. tablets, the use of conventional additives such as fillers, colorants, polymeric binders and the like is contemplated. In general any pharmaceutically acceptable additive which does not interfere with the function of the active compounds can be used. Suitable amounts of active ingredients are, for example, a tablet comprising 0.1 to 60 mg of mirtazapine and typically 0.5 to 500 mg of an NSAID. In a specific example, a tablet comprising 4 mg of mirtazapine and 200 mg of ibuprofen is obtained.

Mirtazapine may be prepared using the method described in US 4,062,848 which is incorporated herein by reference.
Paracetamol and an NSAID may be prepared by any method known in the art. Typically the mentioned compounds are prepared by the methods described in patents concerning these compounds.

The invention is further illustrated by the following examples.

### Example 1

Mirtazapine is formulated in dosing units containing 0.5 mg, 1.5 mg and 4.5 mg mirtazapine.
The dosing units containing 0.5 mg (as tablets) have the composition as indicated in table 1:

**Table 1**

| | Per tablet | Per batch |
|---|---|---|
| Tablet excipients | in mg | In gram |
| Mirtazapine | 0.5 | 7.7 |
| Corn starch | 6.5 | 100.0 |
| Hydroxypropylcellulose | 1.3 | 20.0 |
| Magnesium stearate | 0.4875 | 7.5 |
| Aerosil | 0.975 | 15.0 |
| Lactose 200 M | to 65 mg | To 1000 gram |

For preparation of tablets a 1000 g granulate batch with the composition indicated in table 1 was prepared by premixing the complete amount of mirtazapine (base) with 100 gram of lactose 200 M in a 1 litre glass container for 10 minutes on a Turbula mixer at 22 rotations per minute (rpm). The mixture is sieved through a 150 µm sieve before and after which a further 20 g of lactose 200 M is added. Granulation was performed in a high shear mixer granulator with the rest of the lactose, cornstarch and hydroxypropyl-cellulose. The granulate was dried in a tray vacuum cabinet, classified with a conical screen mill and mixed with aerosil and magnesium stearate. The 65 mg tablets were compressed with a diameter of 5 mm and a radius of convexity of 7.5 mm. Tablets with 1.5 and 4.5 mg mirtazapine were prepared similarly whereby the quantity of lactose was adapted in order to compensate for the increased quantity of mirtazapine.
Dosing units containing, 1.5 mg and 4.5 mg mirtazapine were prepared analogously with compensatory reduction of the amount of lactose 200 Mesh.

### Example 2

A 55 year old female subject, episodically suffering from tension-type headache was unresponsive to treatment with ibuprofen. This drug did not relieve the symptoms when given at the generally recommended dose of 200 mg, nor at the elevated dose of 400 mg.
Addition of 3-4 mg mirtazapine (Remeron®) to 200 mg of ibuprofen, however, results in complete disappearance of the headache. Mirtazapine alone has no effect. It is concluded that in this subject acute treatment of tension-type headache with a combination of a normal dose of ibuprofen and a low dose of mirtazapine is successful and that ibuprofen and mirtazapine act synergistically since neither drug alone has the desired effect.

## Claims

1. A combination comprising paracetamol or a non-steroidal anti-inflammatory drug (NSAID), or a pharmaceutically acceptable salt or solvate thereof, and another drug, **characterised in that** the other drug is mirtazapine, or a pharmaceutically acceptable salt or solvate thereof, optionally in association with one or more pharmaceutically acceptable carriers.

2. The combination according to claim 1, **characterised in that** the combination comprises a pharmaceutical composition which comprises both mirtazapine and paracetamol or an NSAID, optionally in association with one or more pharmaceutically acceptable carriers.

3. The combination according to claim 1 or 2, **characterised in that** the combination is with the NSAID ibuprofen.

4. Use of mirtazapine in combination with paracetamol or an NSAID in the manufacture of a medicament for the treatment of headache.

5. Use of mirtazapine in the manufacture of a medicament, **characterised in that** the medicament is for the treatment of headache, which treatment comprises administration of mirtazapine in combination with paracetamol or an NSAID.

6. The use according to claim 5, **characterised in that** the administration of mirtazapine is with a unit treatment dose comprising between 0.1 and 5 mg mirtazapine.

7. Use of paracetamol or an NSAID in the manufacture of a medicament for the treatment of headache, **characterised in that** the medicament is for administration in combination with mirtazapine.

8. The uses according to any one of claim 4 to 7, **characterised in that** the headache is tension-type headache.

9. A patient pack for the treatment of headache comprising means for administration of metered dose units in combination with packaging material suitable for said dose units, **characterised in that** the patient pack comprises mirtazapine and comprises paracetamol or an NSAID and optionally, said packaging material is including means to help a recipient using the dose units most suitably for the treatment of headache.

10. A patient pack according to claim 9, **characterised in that** at least one of the metered dose units comprises mirtazapine in an amount between 0.1 and 5 mg.

## Patentansprüche

1. Eine Kombination umfassend Paracetamol oder ein nichtsteroidales entzündungshemmendes Arzneimittel (NSAID) oder ein pharmazeutisch verträgliches Salz oder Solvat davon, und ein anderes Arzneimittel, **dadurch gekennzeichnet, dass** das andere Arzneimittel Mirtazapine oder ein pharmazeutisch verträgliches Salz oder Solvat davon ist, optional zusammen mit einem oder mehreren pharmazeutisch verträglichen Trägern.

2. Kombination nach Anspruch 1, **dadurch gekennzeichnet, dass** die Kombination eine pharmazeutische Zusammensetzung umfasst, die sowohl Mirtazapine und Paracetamol oder ein NSAID umfasst, optional zusammen mit einem oder mehreren pharmazeutisch verträglichen Trägern.

3. Kombination nach Anspruch 1 oder 2, **dadurch gekennzeichnet, dass** die Kombination mit dem NSAID Ibuprofen vorliegt.

4. Verwendung von Mirtazapine in Kombination mit Paracetamol oder einem NSAID bei der Herstellung eines Medikamentes für die Behandlung von Kopfschmerzen.

5. Verwendung von Mirtazapine bei der Herstellung eines Medikamentes, **dadurch gekennzeichnet, dass** das Medikament für die Behandlung von Kopfschmerzen vorgesehen ist, welche Behandlung die Verabreichung von Mirtazapine in Kombination mit Paracetamol oder einem NSAID umfasst.

6. Verwendung nach Anspruch 5, **dadurch gekennzeichnet, dass** die Verabreichung von Mirtazapine eine Behandlungsdosiseinheit zwischen 0,1 und 5 Milligramm Mirtazapine umfasst.

7. Verwendung von Paracetamol oder einem NSAID bei der Herstellung eines Medikamentes zur Behandlung von Kopfschmerzen, **dadurch gekennzeichnet, dass** das Medikament zur Verabreichung in Kombination mit Mirtazapine vorgesehen ist.

8. Verwendung nach einem der vorstehenden Ansprüche 4 bis 7, **dadurch gekennzeichnet, dass** die Kopfschmerzen Spannungskopfschmerzen sind.

9. Eine Patientenpackung für die Behandlung von Kopfschmerzen mit Mitteln zur Verabreichung von abgemessenen Dosiseinheiten in Kombination mit Verpackungsmaterial, welches für die besagten Dosiseinheiten geeignet ist, **dadurch gekennzeichnet, dass** die Patientenpackung Mirtazapine umfasst und Paracetamol oder ein NSAID umfasst, und optional dass das besagte Verpackungsmaterial Mittel umfasst, um einem Empfänger zu helfen, die für die Behandlung der Kopfschmerzen geeignetsten Dosiseinheiten zu benutzen.

10. Patientenpackung nach Anspruch 9, **dadurch gekennzeichnet, dass** mindestens eine der abgemessenen Dosiseinheiten Mirtazapine in einer Menge zwischen 0,1 und 5 mg umfasst.

## Revendications

1. Une association comprenant le paracétamol ou un médicament anti-inflammatoire non-stéroïdien (AINS), ou un sel ou un solvate de celui-ci pharmaceutiquement acceptable, et un autre médicament, **caractérisé en ce que** ce dernier est la mirtazapine, ou un sel ou un solvate de celle-ci pharmaceutiquement acceptable, le cas échéant, en association avec un ou plusieurs excipient(s) pharmaceutiquement acceptable(s).

2. L'association selon la revendication 1, **caractérisée en ce que** l'association comprend une composition pharmaceutique qui comprend à la fois la mirtazapine et le paracétamol ou un AINS, le cas échéant, en association avec un ou plusieurs excipients pharmaceutiquement acceptable(s).

3. L'association selon la revendication 1 ou 2, **caractérisée en ce que** l'association est réalisée avec l'ibuprofène comme AINS.

4. Utilisation de la mirtazapine en association avec le paracétamol ou un AINS dans la fabrication d'un médicament pour le traitement des maux de tête.

5. Utilisation de la mirtazapine dans la fabrication d'un médicament **caractérisé en ce que** le médicament est pour le traitement des maux de tête, celui-ci comprenant l'administration de la mirtazapine en association avec le paracétamol ou un AINS.

6. L'utilisation selon la revendication 5, **caractérisé en ce que** l'administration de la mirtazapine est réalisée selon une posologie de traitement unitaire comprise entre 0,1 et 5mg de mirtazapine.

7. Utilisation du paracétamol ou d'un AINS dans la fabrication d'un médicament pour le traitement des maux de tête, **caractérisé en ce que** le médicament est destiné à être administré en association avec la mirtazapine.

8. Les utilisations selon l'une quelconque des revendications 4 à 7, **caractérisées en ce que** les maux de tête sont des maux de tête de type tension.

9. Une trousse pour patient destiné au traitement de maux de tête comprenant les moyens permettant l'administration d'unités posologiques mesurées en association avec le matériau d'emballage approprié pour lesdites unités posologiques, **caractérisée en ce que** la trousse pour patient comprend de la mirtazapine et comprend du paracétamol ou un AINS et, le cas échéant, ledit matériau d'emballage inclut des moyens pour aider un patient receveur à utiliser des unités posologiques de façon la plus appropriée pour le traitement des maux de tête.

10. Une trousse pour patient selon la revendication 9, **caractérisée en ce qu'**au moins une des unités posologiques mesurées comprend la mirtazapine selon une quantité comprise entre 0,1 et 5mg.
